# EUROPÄISCHE PATENTANMELDUNG

(11) **EP 4 344 764 A1**
(43) Veröffentlichungstag der Anmeldung: **03.04.2024**
(21) Anmeldenummer: 22020469.7
(22) Anmeldetag: 29.09.2022
(51) Int. Cl.: B01D 53/00, B01D 53/26, B01D 5/00, C07C 5/32

(54) **VERFAHREN UND ANLAGE ZUR TRENNTECHNISCHEN BEARBEITUNG EINES WASSERSTOFFHALTIGEN EINSATZSTROMS**

(71) Anmelder: Linde GmbH, 82049 Pullach (DE)
(72) Erfinder: Eberl, Christian, 82049 Pullach (DE)
(74) Vertreter: Fischer, Werner

(57) **Zusammenfassung**

Es wird ein Verfahren (200) zur trenntechnischen Bearbeitung eines zumindest Wasserstoff, Wasser und Kohlenwasserstoffe mit drei und vier Kohlenstoffatomen enthaltenden Einsatzstroms (201) vorgeschlagen, bei dem der Einsatzstrom (201) einem ersten Wärmetausch (220) gegen ein oder mehrere Kältemittel (271) unterworfen und bei dem ersten Wärmetausch (220) unter Erhalt einer ersten Gasphase, einer wässrigen Flüssigphase, und einer ersten organischen Flüssigphase abgekühlt und teilkondensiert wird, unter Verwendung der ersten Gasphase oder eines Teils hiervon ein Tieftemperaturtrenneinsatzstrom (205) gebildet wird, der Tieftemperaturtrenneinsatzstrom (205) einem zweiten Wärmetausch (110) in einer Tieftemperaturtrennung (260) unterworfen und bei dem zweiten Wärmetausch unter Erhalt einer zweiten Gasphase und einer zweiten organischen Flüssigphase abgekühlt und teilkondensiert wird, und die zweite Gasphase oder ein Teil hiervon und die zweite Flüssigphase oder ein Teil hiervon bei dem zweiten Wärmetausch gegen den gegen den Tieftemperaturtrenneinsatzstrom (205) erwärmt wird. Eine entsprechende Anlage ist ebenfalls Gegenstand der vorliegenden Erfindung.

## Beschreibung

Die Erfindung betrifft ein Verfahren sowie eine Anlage zur trenntechnischen Bearbeitung eines zumindest Wasserstoff, Wasser und einen oder mehrere Kohlenwasserstoffe enthaltenden Einsatzstroms.

### Hintergrund der Erfindung

Propylen (Propen) wird herkömmlicherweise überwiegend durch Dampfspalten (engl. Steam Cracking) von Kohlenwasserstoffeinsätzen und weitere Umwandlungsverfahren im Zuge von Raffinerieprozessen hergestellt. In diesen Fällen stellt Propylen ein Nebenprodukt dar, das allerdings nur in vergleichsweise geringen Mengen anfällt. Aufgrund des zunehmenden Bedarfs an Propylen, insbesondere für Polypropylenproduktion, wird daher auch die Propandehydrierung eingesetzt.

Die (katalytische) Propandehydrierung ist ein allgemein bekanntes Verfahren der petrochemischen Industrie und wird beispielsweise im Artikel "Propene" in Ullmann's Encyclopedia of Industrial Chemistry, Onlineausgabe 16. September 2013, DOI: 10.1002/14356007.a22_211 .pub3, insbesondere Abschnitt 3.3.1, "Propane Dehydrogenation", beschrieben.

Bei der Propandehydrierung handelt es sich um eine endotherme Gleichgewichtsreaktion, die im Allgemeinen an Edel- oder Schwermetallkatalysatoren, umfassend beispielsweise Platin oder Chrom, durchgeführt wird. Die Dehydrierungsreaktion ist ausgesprochen selektiv. Für kommerziell verfügbare Prozesse werden Gesamtausbeuten von ca. 90% genannt. Ungeachtet dieser hohen Selektivität entstehen neben dem abgespaltenen Wasserstoff jedoch typischerweise auch geringere Mengen an Kohlenwasserstoffen mit einem, zwei sowie vier und mehr als vier Kohlenstoffatomen als Nebenprodukte. Diese Nebenprodukte sowie bei der Propandehydrierung nicht umgesetztes Propan müssen zur Gewinnung eines Propylenprodukts abgetrennt werden.

Dampfspaltverfahren und Raffinerieprozesse, in denen Propylen gebildet wird, sind ebenfalls umfangreich in der Literatur beschrieben, beispielsweise im Artikel "Ethylene" in Ullmann's Encyclopedia of Industrial Chemistry, Onlineveröffentlichung 15. April 2009, DOI: 10.1002/14356007.a10_045.pub3 , und im Artikel "Oil Refining" in Ullmann's Encyclopedia of Industrial Chemistry, Onlineveröffentlichung 15. Januar 2007, DOI: 10.1002/14356007.a18_051.pub2 .

Auch in Dampfspaltverfahren und Raffinerieprozessen werden Propylen enthaltende Komponentengemische gebildet, die zur Gewinnung eines Propylenprodukts entsprechend bearbeitet werden müssen. Die vorliegende Erfindung eignet sich grundsätzlich für alle Verfahren und Anlagen, in denen Komponentengemische gebildet werden, die Propylen sowie insbesondere tiefer als Propylen siedende Komponenten enthalten.

In kommerziellen Anlagen zur Propandehydrierung werden bei der Bearbeitung eines entsprechenden Komponentengemischs nach einer entsprechenden Vorbereitung, beispielsweise einer Verdichtung und Kohlendioxidentfernung, typischerweise zunächst Kohlenwasserstoffe mit zwei Kohlenstoffatomen und leichter siedende Verbindungen von Kohlenwasserstoffen mit drei und mehr Kohlenstoffatomen kryogen abgetrennt (sogenannte Deethanisierung). Anschließend erfolgt, falls vorhanden, ggf. eine Abtrennung von Kohlenwasserstoffen mit drei Kohlenstoffatomen und die Auftrennung der verbleibenden, überwiegend oder ausschließlich Propan und Propylen enthaltenden Fraktion (sogenannte P-P-Trennung). Vergleichbare Verfahren kommen auch zur Bearbeitung von Komponentengemischen zum Einsatz, die in Dampfspaltverfahren und Raffinerieprozessen gebildet werden.

Die vorliegende Erfindung stellt sich die Aufgabe der trenntechnischen Bearbeitung von propylenhaltigen Gasgemischen, die aus der Propandehydrierung stammen können, jedoch nicht notwendigerweise stammen, und die Kohlenwasserstoffe mit drei und vier Kohlenstoffatomen und leichtere Komponenten aufweisen, wie unten erläutert.

### Offenbarung der Erfindung

Vor diesem Hintergrund schlägt die vorliegende Erfindung Verfahren und Anlagen gemäß den unabhängigen Patentansprüchen vor. Vorteilhafte Ausgestaltungen und Weiterbildungen sind Gegenstand der abhängigen Patentansprüche sowie der nachfolgenden Beschreibung.

Fluide beliebiger Art werden hier generell als "reich" an einer oder mehreren enthaltenen Komponenten bezeichnet, wenn der Gehalt an der einen oder den mehreren Komponenten wenigstens 50%, 60%, 70%, 80%, 90%, 95%, 99% oder 99,5% auf molarer, Gewichts- oder Volumenbasis beträgt. Sie werden hingegen als "arm" an der einen oder den mehreren Komponenten bezeichnet, wenn der Gehalt an der einen oder den mehreren Komponenten höchstens 30%, 20%, 10%, 5%, 1% oder 0,5% auf molarer, Gewichts- oder Volumenbasis beträgt. Sie können im hier verwendeten Sprachgebrauch "angereichert" oder "abgereichert" an einer oder mehreren Komponenten sein, wobei sich diese Begriffe auf einen entsprechenden Gehalt in einem Ausgangsfluid beziehen, aus dem das jeweilige Fluid gebildet wurde. Das Fluid wird als angereichert bezeichnet, wenn es zumindest den 2-fachen, 5-fachen, 10-fachen, 100-fachen oder 1.000-fachen Gehalt der einen oder der mehreren Komponenten, bezogen auf das Ausgangsfluid, aufweist. Das Fluid wird hingegen als abgereichert bezeichnet, wenn es höchstens den 0,5-fachen, 0,1-fachen, 0,01-fachen oder 0,001-fachen Gehalt der einen oder der mehreren Komponenten, bezogen auf das Ausgangsfluid, aufweist. Ein "überwiegend" eine oder mehrere Komponenten enthaltendes Fluid ist reich an dieser oder diesen im Sinne der obigen Definition.

Ein Fluid (die Bezeichnung Fluid wird stellvertretend auch für entsprechende Ströme, Fraktionen usw. verwendet) ist im hier verwendeten Sprachgebrauch von einem anderen Fluid (das hier auch als Ausgangsfluid bezeichnet wird) abgeleitet oder aus einem solchen Fluid gebildet, wenn es zumindest einige in dem Ausgangsfluid enthaltene oder aus diesem erhaltene Komponenten aufweist. Ein in diesem Sinne abgeleitetes bzw. gebildetes Fluid kann aus dem Ausgangsfluid durch Abtrennen oder Abzweigen eines Anteils oder einer oder mehrerer Komponenten, Anreichern oder Abreichern bezüglich einer oder mehrerer Komponenten, chemisches oder physikalisches Umsetzen einer oder mehrerer Komponenten, Erwärmen, Abkühlen, Druckbeaufschlagen und dergleichen erhalten bzw. gebildet werden. Ein Stoffstrom kann auch beispielsweise einfach dadurch gebildet werden, dass er aus einem Speicherbehälter abgezogen bzw. von einem anderen Stoffstrom abgetrennt wird.

Nachfolgend werden zur Charakterisierung von Drücken und Temperaturen die Begriffe "Druckniveau" und "Temperaturniveau" verwendet, wodurch zum Ausdruck gebracht werden soll, dass Drücke und Temperaturen nicht in Form exakter Druck- bzw. Temperaturwerte vorliegen müssen. Ein Druckniveau oder Temperaturniveau kann beispielsweise ±1%, 5%, 10%, 20% oder 50% um einen Mittelwert liegen. Mehrere Druck- und Temperaturniveaus können disjunkte oder überlappende Bereiche darstellen. Dasselbe Druck- bzw. Temperaturniveau kann beispielsweise auch dann noch vorliegen, wenn sich aufgrund von Leitungsverlusten oder Abkühlung Drücke und Temperaturen reduziert haben. Bei hier in bar angegebenen Druckniveaus handelt es sich um Absolutdrücke.

In Ausgestaltungen der vorliegenden Erfindung wird vorgeschlagen, einen Einsatzstrom der bereits zuvor angesprochenen und nachfolgend noch erläuterten Art gegen verdampfendes Kältemittel aus einer zur Anlage gehörenden Kälteanlage, vorzugsweise Propylenkälteanlage, abzukühlen und zum Teil einzukondensieren. Dabei entstehen eine wässrige und eine kohlenwasserstoffreiche flüssige Phase. Die beiden flüssigen Phasen und die verbleibende wasserstoffreiche Gasphase können in einem oder mehreren Abscheidern voneinander getrennt werden. Die wässrige Flüssigphase kann als Abfallstrom zur Anlagengrenze geführt werden.

Die kohlenwasserstoffreiche Phase enthält im wesentlichen Kohlenwasserstoffe mit drei und mehr Kohlenstoffatomen und kann als ein erster Teil einer gewünschten flüssigen Fraktion verwendet werden, die beispielsweise zur Steigerung der Ausbeute in eine Dehydrieranlage zurückgeführt wird.

In einem nachfolgenden Prozessschritt kann Wasserstoffchlorid, falls vorhanden, aus dem Gasstrom entfernt werden. Danach kann evtl. vorhandenes Restwasser in einem Trocknungsschritt, vorzugsweise durch Temperaturwechseladsorption, entfernt werden. Dies ist erforderlich, um das Ausfrieren von Wasser im anschließenden kryogenen Prozessschritt zu verhindern.

In der nachfolgenden kryogenen Trennung (Cold Box) wird der Gasstrom in einem Wärmetauscher gegen kalte Produktströme weiter abgekühlt und enthaltene Kohlenwasserstoffe werden einkondensiert. Die beiden Phasen können in einem Abscheider voneinander getrennt werden. Die kohlenwasserstoffreiche flüssige Phase wird in der Cold Box gegen warmes Einsatzgas angewärmt und kann als zweiter Teil der gewünschten Fraktion mit Kohlenwasserstoffen mit im Wesentlichen drei und vier Kohlenstoffatomen an der Anlagengrenze abgegeben werden. Die Gasphase aus dem Abscheider kann ebenfalls angewärmt und anschließend aufgeteilt werden. Ein Teil kann mit unvermindertem Druck an der Anlagengrenze abgegeben werden. Ein anderer Teil kann in einer oder mehreren Stufen, vorzugsweise in einem Expander, entspannt werden, um Kälte für die kryogene Trennung zur Verfügung zu stellen.

Die entspannte Gasphase kann zur Regeneration der dem kryogenen Teil vorgelagerten Trocknerstation verwendet und schließlich an der Anlagengrenze abgegeben werden. Weiterhin kann der kryogenen Trennung Kälte durch verdampfendes Kältemittel aus einer zur Anlage gehörenden Kälteanlage zur Verfügung gestellt werden.

Somit wird im Rahmen der vorliegenden Erfindung ein Verfahren zur trenntechnischen Bearbeitung eines zumindest Wasserstoff, Wasser und Kohlenwasserstoffe mit drei und vier Kohlenstoffatomen enthaltenden Einsatzstroms vorgeschlagen, bei dem der Einsatzstrom einem ersten Wärmetausch gegen ein oder mehrere Kältemittel unterworfen und bei dem ersten Wärmetausch unter Erhalt einer ersten Gasphase, einer wässrigen Flüssigphase, und einer ersten organischen Flüssigphase abgekühlt und teilkondensiert wird, unter Verwendung der ersten Gasphase oder eines Teils hiervon ein Tieftemperaturtrenneinsatzstrom gebildet wird, der Tieftemperaturtrenneinsatzstrom einem zweiten Wärmetausch in einer Tieftemperaturtrennung unterworfen und bei dem zweiten Wärmetausch unter Erhalt einer zweiten Gasphase und einer zweiten organischen Flüssigphase abgekühlt und teilkondensiert wird, und die zweite Gasphase oder ein Teil hiervon und die zweite Flüssigphase oder ein Teil hiervon bei dem zweiten Wärmetausch gegen den Tieftemperaturtrenneinsatzstrom erwärmt wird. Der Einsatzstrom kann auf einem Druckniveau von 25 bis 35 bar, beispielsweise ca. 30 bar, und einem Temperaturniveau von 25 bis 50°C, beispielsweise ca. 42°C, bereitgestellt werden.

Die vorliegende Erfindung schafft auf diese Weise ein Verfahren, in dem ein Wasserstoff und Kohlenwasserstoffe (im Wesentlichen mit einem bis vier Kohlenstoffatomen, insbesondere mit drei und vier Kohlenstoffatomen) enthaltender Stoffstrom auf besonders vorteilhafte Weise in eine wasserstoffreiche Fraktion und eine an Kohlenwasserstoffen mit drei und vier Kohlenstoffatomen reiche Fraktion aufgetrennt werden kann. Dabei können besonders hohe Ausbeuten erzielt und eine Gasfraktion zu einem Teil bei ausgesprochen hohem Druck abgegeben werden. Auch eine Kohlenwasserstoffe mit drei und vier Kohlenstoffatomen enthaltende Flüssigfraktion kann mit hohem Druck an die Anlagengrenze geführt bzw. rückgeführt werden, so dass ein Pumpeneinsatz reduziert werden kann. Die Investitions- und Betriebskosten (im Wesentlichen der Bedarf an externer Kälte) können im Rahmen der vorliegenden Erfindung und ihrer Ausgestaltungen minimiert werden.

Die erste Gasphase ist gegenüber dem Einsatzstrom insbesondere an den Kohlenwasserstoffen mit drei und vier Kohlenstoffatomen abgereichert und an Wasserstoff angereichert, und die zweite Gasphase ist gegenüber der ersten Gasphase insbesondere an den Kohlenwasserstoffen mit drei und vier Kohlenstoffatomen weiter abgereichert und an Wasserstoff weiter angereichert. Die erste und die zweite Flüssigphase gemeinsam können insbesondere zumindest 90% oder 95% der Kohlenwasserstoffe mit drei und mehr Kohlenstoffatomen aus dem Einsatzstrom enthalten. Die Ausbeute an Kohlenwasserstoffen mit vier Kohlenstoffatomen kann insbesondere bei mehr als 99,5% und die Ausbeute an Kohlenwasserstoffe mit drei Kohlenstoffatomen bei mehr als 96% liegen.

In Ausgestaltungen der vorliegenden Erfindung wird, wie bereits angesprochen, ein Teil der zweiten Gasphase nach dem Erwärmen in dem zweiten Wärmetausch einer Entspannung unterworfen und nach der Entspannung erneut dem zweiten Wärmetausch unterworfen und dort erwärmt werden. Eine Entspannung kann auch mehrfach durchgeführt werden, bis das gewünschte Druckniveau erreicht ist. So kann beispielsweise eine Entspannung von 30 auf 16 bar in einem ersten Expander, eine Zwischenanwärmung im Zuge des zweiten Wärmetauschs, und dann eine Entspannung von 16 auf 10 bar in einem zweiten Expander erfolgen.

Der Teil der Gasphase, der nach dem Erwärmen in dem zweiten Wärmetausch einer Entspannung unterworfen und nach der Entspannung erneut dem zweiten Wärmetausch unterworfen und dort erwärmt wird, kann nach dem erneuten zweiten Wärmetausch insbesondere als Regeneriergas in einem stromauf der Tieftemperaturtrennung angeordneten adsorptiven Wasserentfernungsschritt verwendet und auf diese Weise vorteilhaft genutzt werden.

Der zweite Wärmetausch kann insbesondere bei 25 bis 30 bar Absolutdruck durchgeführt werden, einem Druckniveau, auf dem auch die zweite Flüssigphase und der nicht entspannte Teil der zweiten Gasphase abgegeben werden können. Insbesondere kann es sich hierbei um Druckniveaus von 27,4 bar Absolutdruck bzw. 28,7 bar Absolutdruck handeln. Die Entspannung kann insbesondere auf ein Druckniveau von 5 bis 10 bar Absolutdruck, insbesondere ca. 8 bar Absolutdruck, durchgeführt werden. Die Entspannung kann, wie bereits erwähnt, in mehreren Stufen über mehrere Expander mit oder ohne Zwischenanwärmung im Zuge des zweiten Wärmetauschs erfolgen.

In Ausgestaltungen der vorliegenden Erfindung werden die erste an Kohlenwasserstoffen reiche Flüssigphase oder ein Teil hiervon und die zweite an Kohlenwasserstoffen reiche Flüssigphase oder ein Teil hiervon vereinigt und in einen zur Bereitstellung des Einsatzstroms verwendeten Verfahrensschritt zurückgeführt. Der Einsatzstrom kann insbesondere einer Alkandehydrierung entnommen werden und die erste, an Kohlenwasserstoffen reiche, bzw. organische Flüssigphase und/oder die zweite, an Kohlenwasserstoffen reiche, bzw. organische Flüssigphase oder jeweils ein Teil hiervon können zu der Alkandehydrierung zurückgeführt werden.

In Ausgestaltungen der Erfindung kann der Einsatzstrom 40 bis 80%, insbesondere 50 bis 70%, Wasserstoff, 0 bis 20%, insbesondere 5 bis 15%, Stickstoff, 0,5 bis 20%, insbesondere 0,5 bis 5%, Kohlenwasserstoffe mit drei Kohlenstoffatomen und 0,5 bis 40%, insbesondere 0,5 bis 30%, Kohlenwasserstoffe mit vier Kohlenstoffatomen und 0 bis 10%, insbesondere 0 bis 5%, einer oder mehrerer weiterer Komponenten enthalten, wobei die Mengenangaben jeweils einen Volumenanteil bezeichnen und sich zu 100% ergänzen. Ein Beispiel für eine entsprechende Gaszusammensetzung ist unten angegeben.

In Ausgestaltungen der vorliegenden Erfindung können in dem zweiten Wärmetausch ein oder mehrere Kältemittel verwendet werden, wobei das eine oder die mehreren Kältemittel, die in dem ersten Wärmetausch verwendet werden, und das eine oder die mehreren Kältemittel, die in dem zweiten Wärmetausch verwendet werden, aus einem gemeinsamen Kältekreislauf entnommen werden. Auf diese Weise gestaltet sich das vorgeschlagene Verfahren als besonderes günstig hinsichtlich der Erstellungs- und Betriebskosten.

Eine Anlage zur trenntechnischen Bearbeitung eines zumindest Wasserstoff, Wasser und Kohlenwasserstoffe mit drei und vier Kohlenstoffatomen enthaltenden Einsatzstroms ist ebenfalls Gegenstand der Erfindung. Die Anlage ist dafür eingerichtet, den Einsatzstrom einem ersten Wärmetausch gegen ein oder mehrere Kältemittel zu unterwerfen und beim ersten Wärmetausch unter Erhalt einer ersten Gasphase, einer wässrigen Flüssigphase, und einer ersten organischen Flüssigphase abzukühlen und teilzukondensieren, unter Verwendung der ersten Gasphase oder eines Teils hiervon einen Tieftemperaturtrenneinsatzstrom zu bilden, den Tieftemperaturtrenneinsatzstrom einem zweiten Wärmetausch in einer Tieftemperaturtrennung zu unterwerfen und bei dem zweiten Wärmetausch unter Erhalt einer zweiten Gasphase und einer zweiten organischen Flüssigphase abzukühlen und teilzukondensieren, und die zweite Gasphase oder einen Teil hiervon und die zweite Flüssigphase oder einen Teil hiervon bei dem zweiten Wärmetausch gegen den Tieftemperaturtrenneinsatzstrom zu erwärmen.

Zu weiteren Merkmalen und Vorteilen einer entsprechenden Anlage und Ausgestaltungen hiervon sei auf die obigen Erläuterungen betreffend das erfindungsgemäß vorgeschlagene Verfahren und seine Ausgestaltungen ausdrücklich verwiesen, da diese hierfür in gleicher Weise gelten.

Entsprechendes gilt auch für eine Anlage, die gemäß einer Ausgestaltung der Erfindung dazu eingerichtet ist, ein Verfahren gemäß einer beliebigen Ausgestaltung der vorliegenden Erfindung durchzuführen.

Weitere Vorteile und Ausgestaltungen der Erfindung werden im Folgenden unter Bezugnahme auf die beiliegende Zeichnung erläutert.

### Kurze Beschreibung der Zeichnung

Ausführungsformen der Erfindung werden nachfolgend rein beispielhaft unter Bezugnahme auf die beigefügte Zeichnung beschrieben, wobei
Figur 1 Aspekte einer Ausgestaltung der Erfindung in Form eines stark vereinfachten Blockdiagramms veranschaulicht, und
Figur 2 ein Verfahren gemäß einer Ausgestaltung der Erfindung in Form eines vereinfachten Blockdiagramms veranschaulicht.

### Ausführungsformen der Erfindung

Die nachfolgend beschriebenen Ausführungsformen werden lediglich zu dem Zweck beschrieben, den Leser beim Verständnis der beanspruchten und zuvor erläuterten Merkmale zu unterstützen. Sie stellen lediglich repräsentative Beispiele dar und sollen hinsichtlich der Merkmale der Erfindung nicht abschließend und/oder beschränkend betrachtet werden. Es versteht sich, dass die zuvor und nachfolgend beschriebenen Vorteile, Ausführungsformen, Beispiele, Funktionen, Merkmale, Strukturen und/oder anderen Aspekte nicht als Beschränkungen des Umfangs der Erfindung, wie er in den Ansprüchen definiert ist, oder als Beschränkungen von Äquivalenten zu den Ansprüchen zu betrachten sind, und dass andere Ausführungsformen verwendet und Änderungen vorgenommen werden können, ohne vom Umfang der beanspruchten Erfindung abzuweichen.

Unterschiedliche Ausführungsformen der Erfindung können weitere zweckmäßige Kombinationen der beschriebenen Elemente, Komponenten, Merkmale, Teile, Schritte, Mittel usw. umfassen, aufweisen, aus ihnen bestehen oder im Wesentlichen aus ihnen bestehen, auch wenn solche Kombinationen hier nicht spezifisch beschrieben sind. Darüber hinaus kann die Offenbarung andere Erfindungen umfassen, die gegenwärtig nicht beansprucht sind, die aber in Zukunft beansprucht werden können, insbesondere wenn sie vom Umfang der unabhängigen Ansprüche umfasst sind.

Hier verwendete Bezugnahmen auf Anlagenkomponenten gelten sinngemäß entsprechend auf in diesen bzw. durch diese Anlagenkomponenten durchgeführte Verfahrensschritte, so dass für Verfahrensschritte und entsprechende Anlagenkomponenten identische Bezugszeichen verwendet werden.

Es sei darauf hingewiesen, dass in den Figuren nicht notwendigerweise sämtliche Merkmale der Erfindung dargestellt sind, da die Figuren lediglich der Veranschaulichung und dem besseren Verständnis, nicht jedoch zur abschließenden Darstellung der Erfindung dienen sollen. Zudem können auch Merkmale gezeigt sein, die nicht in sämtlichen Ausgestaltungen der Erfindung notwendigerweise zur Anwendung kommen.

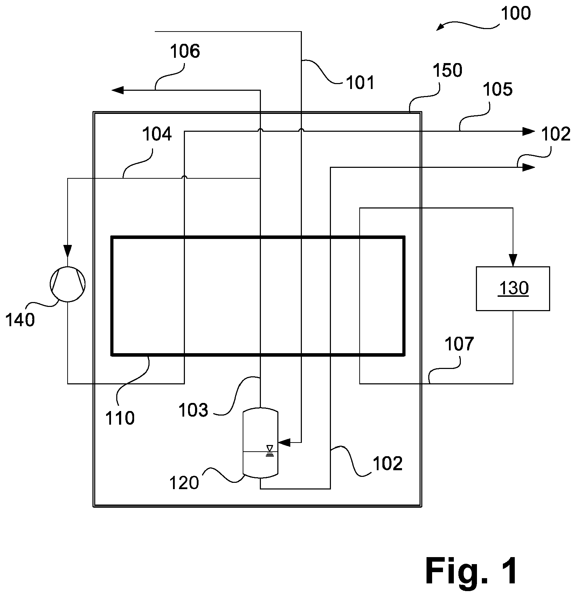

In Figur 1 sind Aspekte einer Ausgestaltung der Erfindung anhand eines stark vereinfachten Blockdiagramms dargestellt und insgesamt mit 100 bezeichnet. Beispielsweise kann die Ausgestaltung 100 als eine Anlage zur trenntechnischen Bearbeitung ausgestaltet sein.

Die Anlage 100 umfasst einen Wärmetauscher 110, einen Separator 120 (auch als Phasentrenner bezeichnet), eine Kältemaschine 130 und einen Expander 140. Im Betrieb der Anlage 100 wird ein Gasstrom, hier auch als Tieftemperaturtrenneinsatzstrom 101 bezeichnet, auf einem ersten Druckniveau in dem Wärmetauscher 110 gekühlt und dabei teilweise kondensiert. Der teilweise kondensierte Tieftemperaturtrenneinsatzstrom 101 wird anschließend dem Separator 120 zugeführt und dort in einen Kondensatstrom 102 und einen zweiten Gasstrom 103 aufgeteilt. Sowohl der Kondensatstrom 102 als auch der zweite Gasstrom 103 werden als Kühlmittel für den Wärmetauscher 110 verwendet und dabei angewärmt. Es handelt sich um die zuvor als "zweiten" Gas- bzw. Flüssigphasen bezeichneten Anteile.

Stromab des Wärmetauschers 110 wird ein erster Teil 104 des zweiten Gasstroms 103 abgezweigt, in dem Expander 140 entspannt, wobei sich der erste Teil 104 des zweiten Gasstroms 103 abkühlt, und dann abermals als Kühlmittel für den Wärmetauscher 110 verwendet. Der dementsprechend erwärmte erste Teil 104 des zweiten Gasstroms 103 wird dann auf einem zweiten Druckniveau, das niedriger als das erste Druckniveau ist, als Niederdruckstrom 105 abgegeben. Ein zweiter Teil 106 des zweiten Gasstroms 103 wird auf dem ersten Druckniveau abgegeben.

Zur Kühlung in dem Wärmetauscher 110 reicht der Druckunterschied zwischen dem ersten und dem zweiten Druckniveau (bzw. der Anteil des ersten Teils 104 des zweiten Gasstroms 103) in der Regel nicht aus. Daher wird zusätzlich ein in der Kältemaschine 130 erzeugter Kältemittelstrom 107 zur Kühlung in dem Wärmetauscher 110 verwendet und dabei in einem entsprechenden Kreislauf geführt.

Wie mit einer Doppellinie dargestellt, können der Wärmetauscher 110 und der Separator 120 in einer Coldbox 150 angeordnet sein. Die gemeinsame Coldbox 150 ist jedoch nicht zwingend erforderlich, so dass auch für sich jeweils einzeln wärmeisolierte Komponenten verwendet werden können.

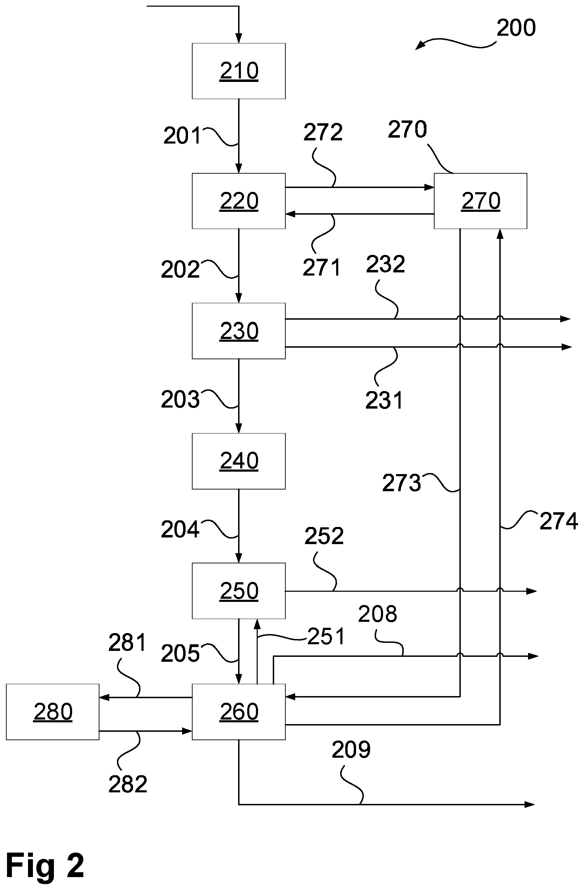

In Figur 2 ist ein Verfahren gemäß einer Ausgestaltung der Erfindung, beispielsweise eine Anlage zur Bearbeitung eines Einsatzstroms aus einer Dehydrierung, gezeigt und insgesamt als 200 bezeichnet.

Die Anlage 200 weist, im Wesentlichen analog zu der soeben beschriebenen Anlage 100, die Komponenten Wärmetauscher und Separator bzw. Phasentrenner (hier zusammenfassend als Tieftemperaturtrennung 260 bezeichnet) sowie Expander 280 und Kältemaschine 270 auf. Ferner umfasst die Anlage 200 eine Vorkühlung 220 (bspw. in Form eines ersten Wärmetauschers), einen ersten Phasentrenner 230, eine Reinigungseinheit 240 und eine Wasserentfernungseinheit bzw. Trocknung 250.

Ferner kann die Anlage 200 einen Reaktor 210 zur Bereitstellung des zu bearbeitenden Einsatzstroms 201 umfassen, beispielsweise eine Vorrichtung, die zur Dehydrierung eines kohlenwasserstoffhaltigen Roheinsatzes eingerichtet ist. Die Bereitstellung des Einsatzstroms 201 kann jedoch auch auf andere Weise erfolgen.

In dem gezeigten Beispiel wird die Kältemaschine 270 zur Bereitstellung sowohl des Kältemittels 273 für die Tieftemperaturtrennung 260, als auch des Kältemittels 271, das in der Vorkühlung 220 verwendet wird, eingesetzt. Entsprechende Rücklaufströme des jeweiligen Kältemittels sind mit 272 bzw. 274 gekennzeichnet. Das Kältemittel 271, 273 kann insbesondere ein C3-Kältemittel sein, also im Wesentlichen aus Kohlenwasserstoffen mit drei Kohlenstoffatomen gebildet sein. In der Vorkühlung 220 wird der (ggf. in dem Reaktor 210 umgesetzte) Einsatzstrom 201 abgekühlt und dabei teilweise kondensiert.

Beispielsweise kann der aus dem Reaktor 210 entnommene Einsatzstrom 201 unsubstituierte sowie einfach oder mehrfach substituierte Alkane und/oder Alkene und/oder Alkine mit 1 bis 5 Kohlenstoffatomen pro Molekül, Wasserstoff, Wasser, Kohlenstoffmonoxid und/oder-dioxid, Inertkomponenten sowie korrosive Verunreinigungen enthalten. Eine beispielhafte relative Zusammensetzung des Einsatzstroms 201 in Volumenanteilen stromab des Reaktors 210 ist in der folgenden Tabelle angegeben. Der Einsatzstrom 201 kann beispielsweise auf einem ersten Druckniveau vorliegen, das im Bereich zwischen 20 bar und 40 bar liegen kann, beispielsweise bei einem Druck von ca. 30 bar.

| | |
|---|---|
| H₂ | 0,629071 |
| N₂ | 0,105787 |
| CO | 0,006200 |
| CO₂ | 0,007926 |
| CH₄ | 0,031705 |
| C₂H₆ | 0,002276 |
| C₂H₄ | 0,002276 |
| C₃H₈ | 0,013655 |
| C₃H₆ | 0,017108 |
| Isobutan | 0,076750 |
| N-Butan | 0,003610 |
| 1-Buten | 0,002433 |
| Isobuten | 0,097076 |
| Pentan | 0,000000 |
| H₂S | 0,000100 |
| NH₃ | 0,000025 |
| 1,3-Butadien | 0,001570 |
| HCl | 0,000001 |
| H2O | 0,002433 |

Der so entstandene abgekühlte Strom 202, der insbesondere zwei Flüssigphasen und eine verbleibende Gasphase umfasst, wird dem ersten Phasentrenner 230 zugeführt, in dem eine wässrige Flüssigphase 231, die im Wesentlichen aus Wasser und darin gelösten Bestandteilen des Einsatzstroms 201 besteht, eine organische ("erste") Flüssigphase 232, die zumindest einen Teil der in dem Einsatzstrom 201 enthaltenen Kohlenwasserstoffe, insbesondere der Kohlenwasserstoffe mit mehr als einem Kohlenstoffatom pro Molekül, umfasst, und eine ("erste") Gasphase gebildet werden.

Unter Verwendung der letzteren wird ein erster Gasstrom 203, der zumindest einen Teil des Wasserstoffes und der Inertkomponenten des Einsatzstroms 201 umfasst, gebildet. Die stromab des ersten Phasentrenners 230 erhaltenen ersten und zweiten Flüssigströme 231, 232 sowie der erste Gasstrom können weiterhin auf dem ersten Druckniveau vorliegen, wobei allfällige Druckverluste das Druckniveau in dem eingangs erläuterten Sinn insgesamt nicht verändern. Der zweite Flüssigstrom, der beispielsweise noch nicht abreagierte gesättigte Kohlenwasserstoffe enthalten kann, kann insbesondere zumindest teilweise wieder zu dem Reaktor 210 zurückgeführt werden, um die Prozessausbeute zu erhöhen.

Stromab dieses ersten Phasentrenners 230 schließt sich eine Reinigung 240 des ersten Gasstroms 203 an, in der zumindest ein Teil der korrosiven Bestandteile des ersten Gasstroms 203, insbesondere HCl, NH3 und/oder H2S aus dem ersten Gasstrom 203 entfernt wird, um einen gereinigten Gasstrom 204 zu erhalten.

Der gereinigte Gasstrom 204 wird anschließend einer Trocknung 250 zugeführt, die beispielsweise als im Wechsel zu betreibende, mehrfache Adsorbereinheit ausgebildet sein kann. Beispielsweise kann eine Temperaturwechseladsorption (engl. temperature swing adsorption, TSA), eine Druckwechseladsorption (engl. pressure swing adsorption, PSA) oder andere geeignete Verfahren zur Trocknung 250 zum Einsatz kommen.

Der derart getrocknete erste Gasstrom 205 wird sodann der Tieftemperaturtrennung 260 zugeführt, die im Wesentlichen analog zu der in Bezug auf Figur 1 beschriebenen trenntechnischen Bearbeitung durchgeführt werden kann, so dass an dieser Stelle zur Vermeidung von unnötigen Wiederholungen auch auf die obigen Ausführungen verwiesen sei. Der zweite Gasstrom bzw. der erste Teil davon (siehe oben; hier als 251 bezeichnet) kann zur zyklischen Regenerierung des Adsorbers der Trocknung 250 verwendet werden. Die während der Trocknung von dem Adsorber aufgenommene Feuchtigkeit wird dementsprechend bei der Regeneration in einem aus dem ersten Teil 251 des zweiten Gasstroms gebildeten Abgasstrom 252 abgeführt.

Der erste Teil des zweiten Gasstroms kann ebenfalls analog zu der in Bezug auf Figur 1 beschriebenen Vorgehensweise zunächst als Kühlmittel für den Wärmetauscher der Tieftemperaturtrennung 260 verwendet werden, als angewärmter Gasstrom 281 in den Expander 280 geführt werden, in dem er unter Abkühlung von dem ersten Druckniveau auf ein zweites, niedrigeres Druckniveau expandiert wird, um dann als Niederdruckstrom 282 abermals als Kühlmittel für den Wärmetauscher der Tieftemperaturtrennung verwendet zu werden. Stromab dieser abermaligen Verwendung als Kühlmittel wird er in dem gezeigten Beispiel sodann als das Regenerationsgas 251 zur Regeneration des Adsorbers der Trocknung 250 verwendet, wie bereits beschrieben. Ein zweiter Teil 208 des zweiten Gasstroms wird hingegen nach seiner Verwendung als Kühlmittel in dem Wärmetauscher der Tieftemperaturtrennung 260 auf dem ersten Druckniveau belassen und als Hochdruckstrom 208 abgegeben. Das zweite Druckniveau kann beispielsweise in einem Bereich von 5 bar bis 15 bar, z.B. um etwa 8 bar, liegen.

Der Tieftemperaturtrennung 260 wird ferner ein Kondensatstrom 209 entnommen, der im Wesentlichen die in dem ersten Gasstrom 203 verbliebenen Kohlenwasserstoffe, insbesondere Kohlenwasserstoffe mit 3 oder 4 Kohlenstoffatomen, umfasst. Dieser Kondensatstrom 209 kann mit Flüssigstrom 232 vereinigt und zum Reaktor 210 zurückgeführt werden.

## Patentansprüche

1. Verfahren (200) zur trenntechnischen Bearbeitung eines zumindest Wasserstoff, Wasser und Kohlenwasserstoffe mit drei und vier Kohlenstoffatomen enthaltenden Einsatzstroms (201), bei dem
- der Einsatzstrom (201) einem ersten Wärmetausch (220) gegen ein oder mehrere Kältemittel (271) unterworfen und bei dem ersten Wärmetausch (220) unter Erhalt einer ersten Gasphase, einer wässrigen Flüssigphase, und einer ersten organischen Flüssigphase abgekühlt und teilkondensiert wird,
- unter Verwendung der ersten Gasphase oder eines Teils hiervon ein Tieftemperaturtrenneinsatzstrom (205) gebildet wird,
- der Tieftemperaturtrenneinsatzstrom (205) einem zweiten Wärmetausch (110) in einer Tieftemperaturtrennung (260) unterworfen und bei dem zweiten Wärmetausch unter Erhalt einer zweiten Gasphase und einer zweiten organischen Flüssigphase abgekühlt und teilkondensiert wird,
- die zweite Gasphase oder ein Teil hiervon und die zweite Flüssigphase oder ein Teil hiervon bei dem zweiten Wärmetausch gegen den Tieftemperaturtrenneinsatzstrom (205) erwärmt wird.

2. Verfahren nach Anspruch 1, bei dem die erste Gasphase gegenüber dem Einsatzstrom (201) an den Kohlenwasserstoffen mit drei und vier Kohlenstoffatomen abgereichert und an dem Wasserstoff angereichert ist, und bei dem die zweite Gasphase gegenüber der ersten Gasphase an den Kohlenwasserstoffen mit drei und vier Kohlenstoffatomen weiter abgereichert und an dem Wasserstoff weiter angereichert ist.

3. Verfahren nach Anspruch 1 oder 2, bei dem die erste und die zweite Flüssigphase gemeinsam zumindest 90% der Kohlenwasserstoffe mit drei und mehr Kohlenstoffatomen aus dem Einsatzstrom (201) enthalten.

4. Verfahren (200) nach einem der vorstehenden Ansprüche, bei dem ein Teil der zweiten Gasphase nach dem Erwärmen in dem zweiten Wärmetausch einer Entspannung unterworfen und nach der Entspannung erneut dem zweiten Wärmetausch unterworfen und dort erwärmt wird.

5. Verfahren (200) nach Anspruch 4, bei dem der Teil der Gasphase, der nach dem Erwärmen in dem zweiten Wärmetausch einer Entspannung unterworfen und nach der Entspannung erneut dem zweiten Wärmetausch unterworfen und dort erwärmt wird, nach dem erneuten zweiten Wärmetausch als Regeneriergas in einem stromauf der Tieftemperaturtrennung (260) angeordneten adsorptiven Wasserentfernungsschritt (250) verwendet wird.

6. Verfahren (200) nach Anspruch 5, wenn dieser von Anspruch 4 abhängt, bei dem die Entspannung auf 5 bis 10 bar Absolutdruck durchgeführt wird.

7. Verfahren (200) nach einem der vorstehenden Ansprüche, bei dem der zweite Wärmetausch auf dem Druckniveau des Einsatzstroms (201), insbesondere bei 25 bis 30 bar Absolutdruck, durchgeführt wird.

8. Verfahren (200) nach einem der vorstehenden Ansprüche, bei dem die erste organische Flüssigphase (232) oder ein Teil hiervon und die zweite organische Flüssigphase (209) oder ein Teil hiervon vereinigt und in einen zur Bereitstellung des Einsatzstroms (201) verwendeten Verfahrensschritt zurückgeführt wird.

9. Verfahren (200) nach einem der vorstehenden Ansprüche, bei dem der Einsatzstrom (201) einer Alkandehydrierung (210) entnommen wird und bei dem die erste organische Flüssigphase und/oder die zweite organische Flüssigphase oder jeweils ein Teil hiervon zu der Alkandehydrierung (210) zurückgeführt werden.

10. Verfahren (200) nach einem der vorstehenden Ansprüche, bei dem der Einsatzstrom 40 bis 80% Wasserstoff, 0 bis 20% Stickstoff, 0,5 bis 20% der Kohlenwasserstoffe mit drei Kohlenstoffatomen und 0,5 bis 40% der Kohlenwasserstoffe mit vier Kohlenstoffatomen und 0 bis 10% einer oder mehrerer weiterer Komponenten enthält, wobei die Mengenangaben jeweils einen Volumenanteil bezeichnen und sich zu 100% ergänzen.

11. Verfahren (200) nach einem der vorstehenden Ansprüche, bei dem in dem zweiten Wärmetausch ein oder mehrere Kältemittel (273) verwendet werden, wobei das eine oder die mehreren Kältemittel (271), die in dem ersten Wärmetausch (220) verwendet werden, und das eine oder die mehreren Kältemittel (273), die in dem zweiten Wärmetausch (110) verwendet werden, aus einem gemeinsamen Kältekreislauf entnommen werden.

12. Anlage zur trenntechnischen Bearbeitung eines zumindest Wasserstoff, Wasser und Kohlenwasserstoffe mit drei und vier Kohlenstoffatomen enthaltenden Einsatzstroms (201), die dafür eingerichtet ist,
- den Einsatzstrom (201) einem ersten Wärmetausch (220) gegen ein oder mehrere Kältemittel (271) zu unterwerfen und beim ersten Wärmetausch (220) unter Erhalt einer ersten Gasphase, einer wässrigen Flüssigphase, und einer ersten organischen Flüssigphase abzukühlen und teilzukondensieren,
- unter Verwendung der ersten Gasphase oder eines Teils hiervon einen Tieftemperaturtrenneinsatzstrom (205) zu bilden,
- den Tieftemperaturtrenneinsatzstrom (205) einem zweiten Wärmetausch (110) in einer Tieftemperaturtrennung (260) zu unterwerfen und bei dem zweiten Wärmetausch unter Erhalt einer zweiten Gasphase und einer zweiten organischen Flüssigphase abzukühlen und teilzukondensieren,
- die zweite Gasphase oder einen Teil hiervon und die zweite Flüssigphase oder einen Teil hiervon bei dem zweiten Wärmetausch gegen den Tieftemperaturtrenneinsatzstrom (205) zu erwärmen.

13. Anlage nach Anspruch 12, die zur Durchführung eines Verfahrens nach einem der Ansprüche 1 bis 11 eingerichtet ist.
